Europäisches Patentamt
European Patent Office
Office européen des brevets

(11) Veröffentlichungsnummer: **0 073 993**
**A2**

# (12) EUROPÄISCHE PATENTANMELDUNG

(21) Anmeldenummer: 82107659.3

(22) Anmeldetag: 21.08.82

(51) Int. Cl.³: **C 07 C 103/46**
C 12 P 13/04, C 07 D 499/12
C 07 D 501/06
//C07C101/28, C07C101/453

(30) Priorität: 03.09.81 DE 3134932

(43) Veröffentlichungstag der Anmeldung: 16.03.83 Patentblatt 83/11

(84) Benannte Vertragsstaaten: AT BE CH DE FR GB IT LI LU NL SE

(71) Anmelder: BAYER AG
Zentralbereich Patente, Marken und Lizenzen
D-5090 Leverkusen 1, Bayerwerk(DE)

(72) Erfinder: Schutt, Hermann, Dr.
Gellert Weg 12
D-5600 Wuppertal 1(DE)

(72) Erfinder: Schmidt, Gunter, Dr.
Pahlkestrasse 63
D-5600 Wuppertal 1(DE)

## (54) Optisch reine alpha-Aminophenylessigsäurederivate, Verfahren zu ihrer Herstellung und ihre Verwendung für die Herstellung von Arzneimitteln.

(57) Die vorliegende Erfindung betrifft neue optisch reine, am α-Phenylring substituierte α-Aminophenylessigsäuren der allgemeinen Formel I

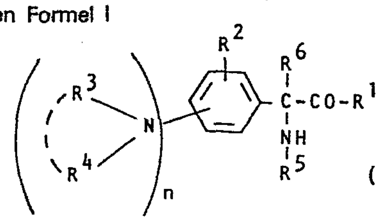

(I)

in welcher $R^1$, $R^2$, $R^3$, $R^4$, $R^5$, $R^6$ und n die in der Beschreibung angegebene Bedeutung haben, eine enzymatisches Verfahren zu ihrer Herstellung durch stereoselektive Spaltung geeigneter racemischer Derivate der genannten Aminosäuren und anschließende Umwandlung in die D- bzw. L-Aminosäuren sowie ihre Verwendung als Zwischenprodukte für die Herstellung von Arzneimitteln.

EP 0 073 993 A2

0073993

BAYER AKTIENGESELLSCHAFT 5090 Leverkusen, Bayerwerk

Zentralbereich
Patente, Marken und Lizenzen Dn/m-c

# Optisch reine α-Aminophenylessigsäurederivate, Verfahren zu ihrer Herstellung und ihre Verwendung für die Herstellung von Arzneimitteln

Die vorliegende Erfindung betrifft neue optisch reine, am α-Phenylring substitutierte α- Aminophenylessigsäuren, ein enzymatisches Verfahren zu ihrer Herstellung durch stereoselektive Spaltung geeigneter racemischer Derivate der genannten Aminosäuren und anschließende Umwandlung in die D- bzw. L-Aminosäuren sowie ihre Verwendung als Zwischenprodukte für die Herstellung von Arzneimitteln.

Die erfindungsgemäßen α-Aminophenylessigsäurederivate entsprechen der allgemeinen Formel (I)

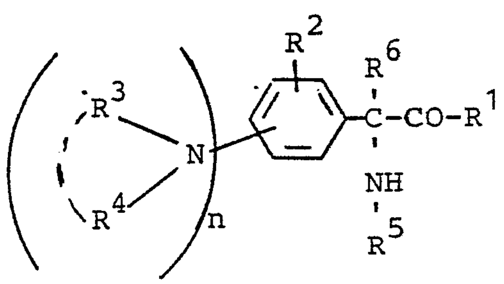

(I)

in welcher

$R^1$ für Hydroxy steht,

$R^2$ für Wasserstoff, Alkyl mit 1 bis 4 Kohlenstoff-

atomen, Trifluormethyl, $C_1$-$C_4$-Alkylmercapto, $C_1$-$C_4$-Alkylsulfinyl, $C_1$-$C_4$-Alkylsulfonyl oder Halogen steht,

$R^3$ und $R^4$ gleich oder verschieden sind und jeweils für Wasserstoff, Alkyl oder Alkenyl mit jeweils bis zu 4 Kohlenstoffatomen, vorzugsweise 1-2 Kohlenstoffatomen stehen, wobei die Alkylreste gegebenenfalls substituiert sind durch Hydroxy, Amino, Cyano oder Alkoxy mit 1 bis 2 Kohlenstoffatomen oder

$R^3$ und $R^4$ gemeinsam mit dem Stickstoffatomen einen 5- bis 7-gliedrigen aliphatischen Ring bilden, der gesättigt oder ungesättigt sein kann und der ein Sauerstoffatom, ein Schwefelatom, eine SO-Gruppierung, eine $SO_2$-Gruppierung, eine N-R"'-Gruppierung, wobei R"' für Wasserstoff oder Alkyl mit 1 bis 2 Kohlenstoffatomen steht, enthalten kann und wobei dieser aliphatische Ring gegebenenfalls noch zusätzlich zwei Stickstoffatome (Triazol) oder drei Stickstoffatome (Tetrazol) enthalten kann,

$R^5$ den Rest einer aliphatischen (bzw. $C_1$-$C_6$) oder araliphatischen (bzw. Benzyl) Carbonsäure oder einer natürlichen oder synthetischen $\alpha$-Aminocarbonsäure.

$R^6$ für Wasserstoff oder Alkyl mit 1 bis 2 Kohlenstoffatomen steht und

n für 0 oder 1 steht, mit der Maßgabe, daß n nicht 0 ist, wenn $R_2$ = H ist.

Die genannten optisch reinen Aminosäuren finden entweder als Ausgangsstoffe für die Herstellung halbsynthetischer

Antibiotika der Penicillin- oder Cephalosporin-Reihe oder als freie Aminosäuren oder Aminosäurederivate zur Behandlung von Entzündungen als Antiphlogistika, z.B. solche nach der DE-OS 2 836 613 Verwendung.

Das erfindungsgemäße Verfahren zur Herstellung der Verbindungen der Formel I ist dadurch gekennzeichnet, daß man auf die racemischen Verbindungen der Formel II

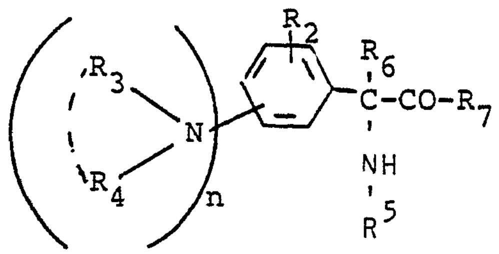

(II)

in der

$R_7$ Alkoxy mit 1-4 C-Atomen oder gegebenenfalls $C_1$-$C_4$-mono- oder disubstituiertes Amino oder den Rest einer natürlichen oder synthetischen Aminosäure bedeutet und in der $R_2$, $R_3$, $R_4$, $R_5$, $R_6$ und n die obengenannte Bedeutung haben,

trägergebundene, proteolytische Enzyme in einem zweiphasigen Medium, bestehend aus Wasser und einem mit Wasser nicht mischbaren Lösungsmittel unter Hydrolyse der L-Derivate zur Carbonsäure einwirken läßt, die D-Derivate der Formel I von den L-Säuren der Formel II in üblicher Weise abtrennt und anschließend auch die D-Derivate in an sich bekannter Weise in die Säuren überführt.

Das erfindungsgemäße Verfahren beruht also darauf, in den L-Verbindungen die Ester- bzw. Amid-Gruppen $CO-R_1$ in die Carboxylgruppe zu überführen, während die D-Verbindung dieser Hydrolyse nicht unterliegt, besonders, wenn die Umsetzung in einem wasserunlöslichen organischen Lösungsmittel durchgeführt wird. Steht die Gewinnung der optisch reinen D-Form im Vordergrund, so ist auf die vollständige Hydrolyse des L-Derivates zu achten; soll hingegen in erster Linie die L-Form optisch rein gewonnen werden, so sollte die Hydrolyse der L-Ester und -Amide nicht vollständig überführt werden, um durch Nebenreaktionen hervorgerufene Hydrolyse der D-Derivate zu vermeiden. Diese Nebenreaktionen laufen vor allem bei Durchführung der Reaktion in alkalischen wäßrigen Lösungen ab.

Die optisch reinen Säuren der Formel I dienen zur Herstellung von optisch reinen Penicillinen und Cephalosporinen, die bisher überwiegend als Epimeren-Gemisch zur Verfügung standen.

Weiterhin können die erfindungsgemäßen Verbindungen auch zur Herstellung bisher nicht bekannter Penicilline und Cephalosporine verwendet werden. Ferner können einige α-Aminophenylessigsäurederivate der allgemeinen Formel I zur Behandlung von Entzündungen verwendet werden.

Chemische Verfahren zur Racematspaltung von Aminosäuren beruhen auf der fraktionierten Kristallisation der Salze der Aminosäuren mit Säuren wie z.B. DL-Camphersulfonsäure (J. P. Greenstein und M. Winitz, Chemistry of Aminoacids, Vol. I (1961) 658ff).

Bei Einsatz von strukturell sehr unterschiedlichen α-Aminophenylessigsäurederivaten ist jedoch nicht zu erwarten, daß mit einer einzigen Säure alle Aminosäuren in die optischen Antipoden getrennt werden können. Es muß daher in jedem speziellen Fall durch langwieriges Probieren die optimal geeignete Säure gefunden werden.

Bei Verwendung der erfindungsgemäß genannten trägergebundenen proteolytischen Enzyme können jedoch alle Derivate der Formel II nach einem Aufarbeitungsschema in die optisch reinen Antipoden getrennt werden.

Dies ist aufgrund der strukturellen Unterschiede der Aminophenylessigsäurederivate nach Formel II überraschend. Die Verwendung nur eines Enzym- und Aufarbeitungssystems für die genannten Aminosäuren ist aber technisch von großer Bedeutung.

Nach Chemical Reviews 46 (1950), 69-153, insbesondere Seiten 119-122 waren die erfindungsgemäß erhaltenen Ergebnisse nicht zu erwarten.

Als Enzyme kommen insbesondere proteolytische Enzyme in Frage, insbesondere Serin- und Sulfhydryl-Proteasen, vorzugsweise Subtilisin (EC 3.4.4.16), α-Chymotrypsin (EC 3.4.4.5), Papain (EC 3.4.4.10.), Ficin oder Bromelain, wobei die ersten beiden einen Serinrest mit aktiven Zentrum der Aminosäurekette haben, während letztere in der Aminosäurekette Cystein als aktives Zentrum aufweisen. Subtilisin ist bevorzugt.

Besonders geeignet sind aus Bacillus subtilis und Bacillus licheniformis isolierte proteolytische Enzyme, die den Waschmitteln zur Entfernung von Proteinresten zugesetzt werden. Diese technischen Enzyme sind vornehmlich unter den Firmennamen Maxatase(R) (Hersteller: Gist-Brocades N.V. Delft/Niederlande), Optimase(R) (Hersteller; Miles-Kali Chemie, Hannover) und Alcalase(R) (Hersteller: Novo Industri AS, Kopenhagen/Dänemark) bekannt.

Die Eigenschaften der proteolytischen Enzyme, insbesondere ihrer biochemischen Wirkungen, sind in folgenden Literaturstellen beschrieben: G. E. Perlmann und L.Lorand, Methods in Enzymology, 19(1979) 199-215; P. Desnuelle, The Enzymes, 4(1960) 93 und G. E. Perlmann und L. Lorand, Methods in Enzymology, 19(1970) 226-244.

Die proteolytischen Enzyme können durch eine kovalente Bindung über einen zur Katalyse nicht essentiellen Lysinrest an den polymeren Träger gekuppelt werden. Die durch kovalente Bindung des Enzyms an den Träger entstehenden trägergebundenen Enzyme verlieren nur sehr langsam nach vielfacher Wiederverwendung ihre Aktivität. Eine weitere Möglichkeit ist, die Adsorption des Enzyms an die Poren eines geladenen Trägers sowie die anschließende Quervernetzung mit Glutardialdehyd.

Als Enzymträger kommen polymere, poröse Träger wie Cellulosen, z.B. DEAE- oder CM-Cellulose, modifizierte

Polyacrylamidgele mit Amino- oder Hydroxylgruppen oder organische Copolymerisate aus Acrylamid, Methacrylaten oder Methacrylamid und Maleinsäureanhydrid nach den DE-OS 22 15 539 und 22 15 687 in Frage.

Das Enzym wird zur Kupplung mit dem polymeren Träger unter optimalen Bedingungen für die Stabilität des Enzyms zur Reaktion gebracht. Bevorzugte Bedingungen für die Kupplung eines proteolytischen Enzyms sind ein pH-Wert von 6-8 und Temperaturen von etwa 20-40°C. Die Effektivität der Kupplung kann durch Messung der enzymatischen Aktivität am Polymer und im Waschwasser bestimmt werden. Das polymere Enzym kann bei Verwendung im Batch-Verfahren leicht durch Semidentation oder Filtration von der Reaktionslösung abgetrennt und mehrfach eingestzt werden. Der Enzymträger kann auch in Säulen gefüllt und in Gegenwart eines Puffersystems von Substratlösung durchströmt werden.

Voraussetzung zur Eignung eines Enzymträgers für das erfindungsgemäße Verfahren ist ein vorheriger Test zur Adsorption der Ausgangs- und Endprodukte. Geeignete Träger dürfen die Ausgangs- und Endprodukte nur in ganz geringem Umfang oder gar nicht adsorbieren. Ein geeigneter Test ist der Adsorptionstest des Substrates mit enzymfreiem Träger und Bestimmung des nicht gebundenen Substrates im Überstand. Träger, die mehr als etwa 5-10 % des Substrates adsorbieren, sind für die vorgesehene Reaktion nicht geeignet.

Als besonders gute Träger für die proteolytischen Enzyme haben sich die verschiedenen Cellulosen, derivatisierten Cellulosen, Amino- und Hydroxylgruppen-haltigen Polyacrylamidgele sowie die durch Amino- oder Hydroxylgruppen modifizierten Anhydridharze erwiesen. Generell können als Träger alle Amino- und Hydroxylgruppen tragenden polymeren Träger eingesetzt werden, die die Ausgangs- und

Endprodukte des erfindungsgemäßen Verfahrens nicht adsorbieren. Der polymere Träger wird nach an sich bekannten Methoden mit Cyanurchlorid (GB-PS 1.302.706, N. L. Smith und H. M. Lehnhoff, Arch. Biochem., 61 (1974) 392-415, T. H. Finlay et al., Arch. Biochem., 87 (1978) 77-90) oder verschiedenen Halogenpyrimidinen nach DE-OS 26 19 521 oder DE-OS 26 19 451 aktiviert.

Die in dem erfindungsgemäßen Verfahren eingesetzten N-Acylester und -Amide der Aminosäuren werden durch Acylieren der entsprechenden Aminosäureester- bzw. -amidhydrochloride mit stöchiometrischen Mengen Säureanhydrid wie Acetanhydrid und anschließende Abtrennung des N-Acylderivates aus der wäßrigen Phase mit organischen Lösungsmitteln wie Chloroform oder Methylenchlorid gewonnen.

Die enzymatische Spaltung erfolgt vorzugsweise bei einer Temperatur von 20-40°C in einem pH-Bereich von 6 bis 8, wobei der pH-Wert vorzugsweise durch Zugabe einer starken Base bei 7,0 konstant gehalten wird. Das Substrat wird als ca. 10 - 20%ige organische Lösung zu dem in Wasser suspendierten Enzym-Träger zugesetzt, wobei der Lösungsmittelanteil, bezogen auf das Gesamtvolumen, maximal 75 - 80 Vol-% betragen kann. Das Reaktionsmedium wird während der enzymatischen Umsetzung intensiv gerührt. Der Verlauf und der Endpunkt der enzymatischen Reaktion kann durch die Neutralisation der entstehenden $H^+$-Ionen bestimmt werden. Die Neutralisation kann sowohl

durch organische als auch durch anorganische Basen erfolgen. Der Verlauf der enzymatischen Racematspaltung kann durch Bestimmung der Drehwerte der organischen und wäßrigen Phase sowie durch Bestimmung der Reaktionsprodukte mittels Hochdruckflüssigkeitschromatographie in organischer und wäßriger Phase direkt bestimmt werden. Als organisches Lösungsmittel für das erfindungsgemäße Verfahren kommen mit Wasser nicht mischbare Lösungsmittel wie Methylenchlorid, Chloroform, Toluol, Benzol, Essigsäureethylester, Petrolether, Methylisobutylketon oder Isobutanol in Frage.

Zahlreiche Substrate und Produkte von Enzymreaktionen sind in Wasser oder Pufferlösungen nur begrenzt löslich. Aus wirtschaftlichen Gründen sollten aber für die technische Anwendung von Reaktionen mit trägergebundenen Enzymen möglichst konzentrierte Substratlösungen eingesetzt werden. Hierbei sollte darauf geachtet werden, daß die Enzymaktivität nicht beeinträchtigt wird.

Nach Beendigung der enzymatischen Reaktion wird nach dem Absitzen des Enzymharzes die organische Phase abgenommen und die wäßrige Reaktionslösung portionsweise noch einmal mit dem zweifachen Volumen am organischen Lösungsmittel extrahiert. Die Extrakte werden vereinigt. Das Enzymharz wird abfiltriert und die verbleibende wäßrige Phase beispielsweise mit Schwefelsäure sauer gestellt und mit Essigsäureethylester extrahiert. Die erhaltenen Verbindungen werden dann auf ihre optische Reinheit überprüft.

Zur Herstellung der D- bzw. L-Aminosäuren werden die Schutzgruppen $R_7$ und $R_5$ in der dem Fachmann geläufigen Weise abgespalten. Geeignete Bedingungen für die Spaltung sind z. B. Behandlung mit 2 N HCl bei etwa 80°C (entsprechend US PS 4 260 684, Beispiel 8).

Beispiel 1

200 g Cellulose Avicel (Merck) werden in einer Lösung aus 500 ml Wasser und 500 ml Dioxan suspendiert und mit 20 g Cyanurchlorid versetzt.

Der pH-Wert wird mit 2 N NaOH zwischen pH 7,0 und 9,0 gehalten. Nach 45 Minuten Rühren wird die aktivierte Cellulose über eine Fritte abgesaugt und in 800 ml Wasser suspendiert. Es werden 25 g Maxatase (Gist-Brocades N.V., Delft/Niederlande) hinzugefügt und es wird 20 Stunden bei Raumtemperatur und pH 7-8 gerührt. Danach wird der Enzymträger über eine Fritte abgesaugt, portionsweise mit destilliertem Wasser gewaschen und zuletzt trocken gesaugt.

Es werden 600 g feuchte Subtilisin-Cellulose erhalten. Aktivität: 284 ATEE (N-Acetyl-L-tyrosinethylester)-Einheiten/g Enzymträger. Gesamtaktivität: 170 400 ATEE-Einheiten, entsprechend 27,2 % der eingesetzten Aktivität.

Beispiel 2

100 g DEAE-Cellulose (DE-52-Cellulose, Fa. Whatmann Ltd., Springfield/England) werden in ähnlicher Weise wie oben beschrieben mit Cyanurchlorid aktiviert. Davon werden 5 g nach Dialyse gegen Wasser lyophilisierte Maxatase oder Alcalase (659 Anson-Einheiten/g, Hersteller Novo AS, Kopenhagen/Dänemark) kovalent gebunden. 140 g feuchtes

DE-52-Cellulose-Subtilisin mit 740 g ATEE-Einheiten/g Enzymträger werden nach Filtration erhalten.

Die gesamte Aktivitätsausbeute betrug 103 600 ATEE-Einheiten entsprechend 16,5 % der eingesetzten Aktivität.

Beispiel 3

20 g mit Aceton gewaschenes Anhydridharz (80 Gew.-% Tetraethylenglykoldimethylcrylat, 10 Gew.-% Methacrylsäure und 10 Gew.-% Maleinsäureanhydrid)werden in 50 ml Wasser suspendiert.

Es werden 40 ml 10 Gew.-%ige Hexamethylendiamin-Lösung bzw. Ethanolamin-Lösung bei pH 7,0 zugegeben und die Suspension über Nacht bei pH 6,2 durch Titration konstant gehalten. Das Aminharz wird abgesaugt. Mit 1 M NaCL-Lösung wird überschüssiges Hexamethylendiamin ausgewaschen. Anschließend wird mit entsalztem Wasser gewaschen.

Das Aminogruppen tragende Anhydridharz wird in 50 ml Wasser und 50 ml Dioxan suspendiert und 1 Stunde bei Raumtemperatur mit 2 g Cyanurchlorid bei pH 5,0 aktiviert. Das Harz wird mit Dioxan und Wasser gewaschen und 20 Stunden bei Raumtemperatur mit 2 g Maxatase bei pH 8,0 umgesetzt.

Es werden 48,2 g feuchtes Harz mit einer Aktivität von 126 ATEE-Einheiten/g Enzymträger erhalten.

Die gesamte Aktivitätsausbeute betrug 6073 ATEE-Einheiten entsprechend 12,1 % der eingesetzten Aktivität.

Beispiel 4

300 g mit Diethylentriamin umgesetztes Polyacrylnitrilharz wird wie oben angegeben im gewichtsmäßigen Verhältnis Träger : Cyanurchlorid (10:1) aktiviert und mit 30 g Maxatase bei 25°C, pH 8,0 und 16 Stunden zur Umsetzung gebracht.

Es werden 288,5 g feuchtes Enzymharz erhalten.

Die proteolytische Aktivität am Träger betrug 81,0 ATEE-Einheiten/g Enzymharz, insgesamt 23.368 ATEE-Einheiten entsprechend 3,1 % der insgesamt eingesetzten Aktivität.

Beispiel 5

Maxatase (Gist-Brocades N.V., Delft/Niederlande) wird über eine mit Sephadex G 50 coarse gefüllte Säule (11 cm x 97 cm; 9,21 Liter) vorgereinigt.

50 g Maxatase werden in 200 ml 0,01 M Na-Phosphatpuffer pH 7,0 suspendiert, zentrifugiert und der Niederschlag verworfen. Die Lösung wird über die mit Sephadex G 50 coarse gefüllte Säule gepumpt und Fraktionen von 250 ml abgenommen.

Die Fraktionen 7-18 werden vereinigt, gegen Wasser dialysiert und anschließend gefriergetrocknet. Ausbeute = 14,12 g, d.h., 28,2 % des Ausgangsgewichtes.

100 g IRC-50-Harz werden in 250 ml 20mM $CaCl_2$-Lösung suspendiert und über Nacht am Titrator auf pH 6,0 titriert.

Es werden unter Rühren 10 g gereinigte Maxatase zugefügt und die Lösung zur Adsorption des Enzyms 18 Stunden mit 1 N NaOH auf pH 6,0 titriert. Das Harz wird abgesaugt, in 300 ml $CaCl_2$-Lösung mit 2,5 % Glutardialdehyd eingerührt und 6 Stunden bei pH 6,0 gehalten. Das Harz wird danach erneut abgesaugt, auf der Nutsche portioniert mit ca. 500 ml 0,1 M Phosphatpuffer pH 7.0 gewaschen und in dem gleichen Puffer unter Zusatz von 0,05 % Toluol bei +4°C gelagert.

Aktivitätsausbeute (Test mit N-Acetyl-DL-phenylglycinmethylester = APME)

Ansatz mit 2,5 % Glutardialdehyd quervernetzt:

| | |
|---|---|
| Aktivität am Harz | = 645 APME-Einheiten |
| | = 17,2 % der eingesetzten Aktivität |
| Filtrate | = 488,6 APME-Einheiten |
| | = 13,0 % der eingesetzten Aktivität |

Das Subtilisin-Harz wurde zur besseren Stabilität mit 7,5 % Glutardialdehyd nachvernetzt:

Ansatz mit 7,5 % Glutardialdehyd quervernetzt:

| | |
|---|---|
| Aktivität am Harz | = 340 APME-Einheiten |
| | = 4,7 % der eingesetzten Aktivität |
| Filtrate | = 964,8 APME-Einheiten |
| | = 13,4 % der eingesetzten Aktivität |

Beispiel 6

Enzymatische Spaltung von N-Formyl-DL-2-chlor-phenylglycinmethylester in Gegenwart von Methylenchlorid sowie anschließende saure Hydrolyse zu D-2-Chlorphenylglycin.

5 g N-Formyl-DL-2-chlor-phenylglycinmethylester werden in einer Emulsion aus 1000 ml Wasser und 1000 ml Methylenchlorid gelöst und mit 210,8 g nach Beispiel 4 hergestelltem Subtilisin-Harz versetzt. Nach 10 Minuten Rühren bei 37°C und pH 7,0, wobei der pH-Wert mit konz. $NH_3$ konstant gehalten wird, wird Rührer abgeschaltet und die organische Phase abgetrennt. Anschließend wird die wäßrige Phase noch zweimal mit 500 ml Methylenchlorid extrahiert. Die vereinigten organischen Phasen werden am Rotationsverdampfer zur Trockene eingedampft.

Die Ausbeute an N-Formyl-D-2-chlor-phenylglycinmethylester beträgt 2,38 g (95,2 % der Theorie)

$$[\alpha]^{25°C}_{578\ nm} = -138,6° \quad (c = 1 \text{ in Methanol})$$

2,38 g N-Formyl-D-2-chlor-phenylglycinmethylester werden in 100 ml 5 %iger HCl gelöst und 30 Stunden bei 85°C hydrolysiert. Anschließend wird die Salzsäure im Rotationsverdampfer abgezogen und das Konzentrat mit 15 %iger Natronlauge auf pH 4,5 eingestellt. Der erhaltene Niederschlag wird abgesaugt, mit destilliertem Wasser und Aceton gewaschen und getrocknet.

Ausbeute an D-2-Chlor-phenylglycin = 1,78 g (82,0 % der Theorie) bezogen auf N-Formyl-D-2-chlor-phenylglycinmethylester

$$[\alpha]^{25°C}_{578\ nm} = -89,1° \quad (c = 1 \text{ in } 1\ N\ HCl)$$

Die wäßrige Phase der enzymatischen Spaltungslösung wird mit 18 %iger HCl auf pH 1.5 eingestellt und die Lösung mit 3 mal jeweil 300 ml Methylisobutylketon ausgeschüttelt. Die vereinigten organischen Phasen werden am Rotationsverdampfer zur Trockene eingedampft. Ausbeute an N-Acetyl-L-2-chlor-phenylglycin (7,2 g = 119,3 % der Theorie)

$$[\alpha]^{25°C}_{578\ nm} = +115° \quad (c = 1 \text{ in Methanol})$$

## Beispiel 7

Enzymatische Spaltung von N-Acetyl-DL-3-chlor-phenylglycinmethylester in Gegenwart von Methylisobutylketon sowie anschließende saure Hydrolyse zu D-3-Chlor-phenylglycin.

13 g N-Acetyl-DL-3-chlor-phenylglycinmethylester werden in einer Emulsion aus 500 ml Wasser und 500 ml Methylisobutylketon gelöst und mit 384 g nach Beispiel 2 hergestelltem Subtilisin-Träger unter Rühren versetzt. Der pH-Wert wird durch Zugabe von 25 %igem Ammoniak mit einer Methrom-Titrationseinheit (pH-Wert E 300 B, Impulsomat E 473 und Dosimat 412, Methrom, Herisau/Schweiz) konstant bei pH 7,0 gehalten. Nach einer Reaktionszeit von 10 Stunden bei 37°C wird der Subtilisin-Träger über eine Fritte abgesaugt und bis zur weiteren Verwendung bei 4°C gelagert.

Die Spaltungslösung wird 3 mal mit je 200 ml Methylisobutylketon extrahiert. Die vereinigten organischen Phasen werden im Rotationsverdampfer zur Trockene eingedampft.

Die Ausbeute an N-Acetyl-D-3-chlor-phenylglycinmethylester beträgt 6,36 g (97,8 % der Theorie)

$$[\alpha]_{578\ nm}^{25°C} = -131,0° \quad (c = 1 \text{ in Methanol})$$

5 g N-Acetyl-D-chlor-phenylglycinmethylester werden in 100 ml 5 %iger HCl gelöst und 18 Stunden bei 85°C hydrolysiert. Die Salzsäure wird am Rotationsverdampfer fast vollständig abgezogen und die Lösung mit 15 %iger Natronlauge auf pH 5,0 eingestellt. Der Niederschlag wird abgesaugt und getrocknet.

Ausbeute an D-3-Chlor-phenylglycin = 2,81 g (73,2 % der Theorie)

$$[\alpha]_{578\ nm}^{25°C} = -112° \quad (c = 1 \text{ in } 1 \text{ N HCl})$$

Die wäßrige Phase der enzymatischen Spaltungslösung wird mit 18 %iger HCl auf pH 1,5 eingestellt und die Lösung mit 3 mal jeweils 300 ml Methylisobutylketon extrahiert. Die vereinigten organischen Phasen werden am Rotationsverdampfer zur Trockene eingedampft.

Ausbeute and N-Acetyl-L-3-chlor-phenylglycin 6,21 g (88,0 % der Theorie)

$$[\alpha]_{578\ nm}^{25°C} = + 155{,}3° \quad (c = 1 \text{ in Methanol})$$

Beispiel 8

Enzymatische Spaltung von N-Acetyl-DL-4-chlor-phenylglycinmethylester in Gegenwart von Methylisobutylketon sowie anschließende saure Hydrolyse zu D-4-chlor-phenylglycin.

15 g N-Acetyl-DL-4-chlor-phenylglycinmethylester werden wie in Beispiel 7 angegeben mit 312 g Subtilisin-Träger, hergestellt nach Beispiel 1, gespalten und die Spaltlösung wie in Beispiel 7 angegeben aufgearbeitet.

Die Ausbeute an N-Acetyl-D-4-chlor-phenylglycinmethylester betrug 5,6 g (74,7 % der Theorie)

$$[\alpha]_{578\ nm}^{25°C} = -155{,}8° \quad (c = 1 \text{ in Methanol})$$

5 g N-Acetyl-D-4-chlor-phenylglycinmethylester werden wie in Beispiel 7 zu D-4-Chlor-phenylglycin hydrolisiert und aufgearbeitet.

Ausbeute an D-4-Chlor-phenylglycin 2,1 g (54,7 % der Theorie)

$$[\alpha]^{25^\circ C}_{578\ nm} = -146^\circ \quad (c = 1 \text{ in } 1\ N\ HCl)$$

Die wäßrige Phase der enzymatischen Spaltlösung wird wie in Beispiel 6 beschrieben zu N-Acetyl-L-4-chlor-phenylglycin aufgearbeitet.

Ausbeute: 7,3 g (119,3 % der Theorie)

$$[\alpha]^{25^\circ C}_{578\ nm} = +155,3^\circ \quad (c = 1 \text{ in Methanol})$$

Beispiel 9

Enzymatische Spaltung von 4-S-Methyl-DL-N-acetyl-phenylglycinmethylester in Gegenwart von Methylenchlorid sowie anschließende saure Hydrolyse zu 4-S-Methyl-D-phenylglycin.

14 g 4-S-Methyl-DL-N-acetyl-phenylglycinester werden in einer Emulsion aus 500 ml dest. Wasser und 500 ml Methylenchlorid gelöst und bei 37°C sowie konstantem pH-Wert von 7,0 (Regulation durch Zugabe von 25 %igem Ammoniak) durch Zugabe von 314 g nach Beispiel 3 herge-

stelltem Subtilisin-Träger gespalten. Nach Abschalten des Rührers werden die wäßrige und organische Phase durch Absaugen von Enzymharz abgetrennt. Die wäßrige Phase wird noch zweimal mit je 500 ml Methylenchlorid extrahiert. Die vereinigten organischen Phasen werden am Rotationsverdampfer zur Trockene eingedampft.

Die Ausbeute an 4-S-Methyl-DL-N-acetyl-phenylglycinmethylester beträgt: 6,12 g (87,4 % der Theorie)

$$[\alpha]^{25°C}_{578\ nm} = -164,3° \quad (c = 1 \text{ in Methanol})$$

5 g 4-S-Methyl-DL-N-acetyl-phenylglycinmethylester werden in 118 ml 5 %iger HCl 18 Stunden bei 85°C hydrolisiert. Die Lösung wurde am Rotationsverdampfer weitgehend eingeengt, mit 15 %iger NaOH auf pH 5,0 eingestellt und der ausgefallene Niederschlag abgesaugt, mit wenig Wasser gewaschen und getrocknet.

Ausbeute an 4-S-Methyl-D-phenylglycin: 3,80 g = 97,5 % der Theorie

$$[\alpha]^{25°C}_{578\ nm} = -97,9° \quad (c = 1 \text{ in } 1 \text{ N HCl})$$

Die wäßrige Phase der enzymatischen Spaltlösung wird mit 18 %iger HCl auf pH 1,5 eingestellt und 3 mal mit jeweils 300 ml Essigsäureethylester extrahiert. Die vereinigten organischen Phasen werden am Rotationsverdampfer zur Trockene eingedampft.

Ausbeute an 4-S-Methyl-L-N-acetyl-phenylglycin 5,35 g = 80,8 % der Theorie

$$[\alpha]^{25°C}_{578\ nm} = +158,1° \quad (c = 1 \text{ in Methanol})$$

Beispiel 10

Enzymatische Spaltung von 4-N-Aminoacetyl-DL-N-acetyl-phenylglycinmethylester in Gegenwart von Methylisobutylketon.

13,9 g 4-N-Aminoacetyl-DL-N-acetyl-phenylglycinmethylester werden in einer Emulsion aus 500 ml dest. Wasser und 250 ml Methylisobutylketon gelöst und wie in Beispiel 6 beschrieben mit 384 g nach Beispiel 4 hergestelltem Subtilisin-Träger gespalten.

Die Aufarbeitung erfolgte wie in Beispiel 6.

Ausbeute an 4-N-Aminoacetyl-D-N-acetyl-phenylglycerinmethylester = 3,19 g = 91,2 % der Theorie

$$[\alpha]^{25°C}_{578\ nm} = -143° \quad (c = 1 \text{ in Methanol})$$

Die wäßrige Phase der enzymatischen Spaltung wird auf pH 1,5 angesäuert und wie in Beispiel 6 beschrieben aufgearbeitet.

Ausbeute an 4-N-Aminoacetyl-L-N-acetyl-phenylglycin = 5,24 g

$$[\alpha]^{25°C}_{578\ nm} = +121° \quad (c = 1 \text{ in Methanol})$$

Patentansprüche:

1. Optisch reine α-Aminophenylessigsäurederivate der allgemeinen Formel I

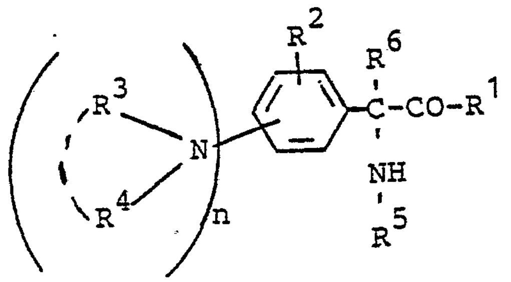

(I)

in welcher

$R^1$ für Hydroxy steht,

$R^2$ für Wasserstoff, Alkyl mit 1 bis 4 Kohlenstoffatomen, Trifluormethyl, $C_1$-$C_4$-Alkylmercapto, $C_1$-$C_4$-Alkylsulfinyl, $C_1$-$C_4$-Alkylsulfonyl oder Halogen steht,

$R^3$ und $R^4$ gleich oder verschieden sind und jeweils für Wasserstoff, Alkyl oder Alkenyl mit jeweils bis zu 4 Kohlenstoffatomen stehen, wobei die Alkylreste gegebenenfalls substituiert sind durch Hydroxy, Amino, Cyano oder Alkoxy mit 1 bis 2 Kohlenstoffatomen oder

$R^3$ und $R^4$ gemeinsam mit dem Stickstoffatom einen 5- bis 7-gliedrigen aliphatischen Ring bilden, der gesättigt oder ungesättigt sein kann und der ein Sauerstoffatom, ein Schwefelatom, eine SO-Gruppierung, eine $SO_2$-Gruppierung, eine N-R"'-Gruppierung, wobei R"' für Wasserstoff oder Alkyl mit 1 bis 2 Kohlenstoffatomen steht, enhalten kann und wobei dieser aliphatische

Ring gegebenenfalls noch zusätzlich zwei Stickstoffatome oder drei Stickstoffatome enthalten kann,

$R^5$ den Rest einer aliphatischen oder araliphatischen Carbonsäure oder einer natürlichen oder synthetischen α-Aminocarbonsäure.

$R^6$ für Wasserstoff oder Alkyl mit 1 bis 2 Kohlenstoffatomen steht und

n für 0 oder 1 steht mit der Maßgabe, daß n nicht 0 ist, wenn $R_2$ = H ist.

2. Verfahren zur Herstellung der Verbindungen nach Anspruch 1, dadurch gekennzeichnet, daß man auf racemische Verbindungen der Formel II

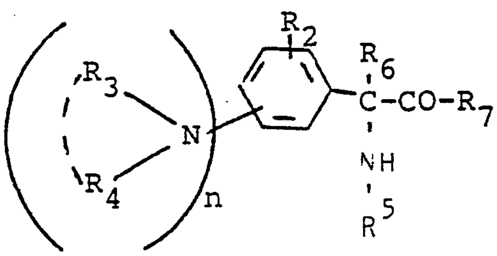

(II)

in der

$R_7$ Alkoxy mit 1-4 C-Atomen, oder gegebenenfalls $C_1$-$C_4$-mono- oder disubstituiertes Amino oder den Rest einer natürlichen oder synthetischen Aminosäure bedeutet und in der

$R_2$, $R_3$, $R_4$, $R_5$, $R_6$ und n die in Anspruch 1 angegebene Bedeutung haben,

trägergebundene, proteolytische Enzyme in einem zweiphasigen Medium, bestehend aus Wasser und einem

mit Wasser nicht mischbaren Lösungsmittel unter Hydrolyse der L-Derivate zur Carbonsäure einwirken läßt, die D-Derivate der Formel I von den L-Säuren der Formel II in üblicher Weise abtrennt und anschließend auch die D-Derivate in an sich bekannter Weise in die Säuren überführt.

3. Verfahren nach Anspruch 2, dadurch gekennzeichnet, daß als Enzym Subtilisin, α-Chymotrypsin, Papain, Ficin oder Bromelain verwendet wird.

4. Verfahren nach den Ansprüchen 2 und 3, dadurch gekennzeichnet, daß die enzymatische Spaltung bei einer Temperatur von 20-40°C durchgeführt wird.

5. Verfahren nach den Ansprüchen 2 - 4, dadurch gekennzeichnet, daß die enzymatische Spaltung bei einem pH von 6-8 durchgeführt wird.

6. Verwendung der Verbindungen gemäß Anspruch 1 bei der Herstellung von Penicillinen oder Cephalosporinen.